# EUROPÄISCHE PATENTSCHRIFT

(11) **EP 0 711 415 B1**
(45) Veröffentlichungstag und Bekanntmachung des Hinweises auf die Patenterteilung: **22.07.1998**
(21) Anmeldenummer: 94905104.9
(22) Anmeldetag: 21.01.1994
(51) Int. Cl.: G01N 33/68, C07K 7/06, C07K 7/08, C12P 21/08, A61K 39/395

(54) **IMMUNOASSAY ZUM NACHWEIS VON KOLLAGEN TYP I ODER KOLLAGENFRAGMENTEN DAVON**
IMMUNOASSAY FOR DETECTING COLLAGEN TYPE I OR COLLAGEN FRAGMENTS THEREOF
IMMUNODOSAGE POUR LA DETECTION DU COLLAGENE TYPE I OU DE FRAGMENTS DE COLLAGENE DE CELUI-CI

(30) Priorität: 28.07.1993 WO PCT/EP93/02010
(43) Veröffentlichungstag der Anmeldung: 15.05.1996
(73) Patentinhaber: BOEHRINGER MANNHEIM GMBH, 68305 Mannheim (DE)
(72) Erfinder: NASER, Werner, D-82362 Weilheim (DE); HUBER, Erasmus, D-86923 Finning (DE); SEIDEL, Christoph, D-82362 Weilheim (DE); ESSIG, Ulrich, D-82152 Planegg (DE)
(86) Internationale Anmeldenummer: EP9400164
(87) Internationale Veröffentlichungsnummer: WO9504282

(56) Entgegenhaltungen:
- EP-A- 0 121 303
- EP-A- 0 298 210
- EP-A- 0 316 306
- EP-A- 0 339 443
- EP-A- 0 465 104
- EP-A- 0 505 210
- WO-A-90/08195
- WO-A-91/09113
- WO-A-93/15107
- DE-A- 4 225 038
- BIOCHEMISTRY Bd. 22 , 1983 Seiten 5213 - 5223 M.P.BERNARD ET AL. 'Nucleotide Sequences of Complementary Deoxyribonucleic Acids for the Pro alpha 1 Chain of Human Type I Procollagen. Statistical Evaluation of Structures That Are Conserved during Evolution.' in der Anmeldung erwähnt
- BIOCHEMISTRY Bd. 9, Nr. 24 , 1970 Seiten 4699 - 4706 E.M.CLICK ET AL. 'Isolation and Characterization of the Cyanogen Bromide Peptides from the alpha 1 and alpha 2 Chains of Human Skin Collagen,' in der Anmeldung erwähnt

## Beschreibung

Die Erfindung betrifft einen Immunoassay zum Nachweis von Kollagen oder Kollagenfragmenten in einer Probe unter Verwendung mindestens eines Antikörpers, der ein synthetisches lineares Peptid erkennt, das einer Sequenz des nichthelikalen C- oder N-terminalen Bereiches von Kollagen entspricht, wobei die Probe denaturiert wird.

Kollagen stellt ein wichtiges Strukturprotein im Bindegewebe von Haut, Knorpel und Knochen dar. Bekannt sind 11 Typen, die jeweils aus drei Ketten bestehen. Jeder Typ setzt sich aus 1 - 3 verschiedenen Ketten zusammen, die als α1, α2 und α3 bezeichnet werden (E. Miller et al. in Methods in Enzymology 144, Structural and Contractile Proteins, ed. L. Cunningham, Academic Press Inc. 1987, p. 3 - 41). Eine charakteristische Eigenschaft des reifen Kollagens bestimmter Gewebe, wie insbesondere Knochen oder Knorpel, ist die Quervernetzung von nebeneinander liegenden Fasern durch Hydroxylysylpyridinolin oder Lysylpyridinolin (D. Fujimoto et al., J. Biochem 83 (1978), 863 - 867; D. Eyre et al., Ann. Rev. Biochem 53 (1984), 717-748 und D. Eyre, Methods in Enzymology 144 (1987), 115-139). Diese Quervernetzungen können als biochemischer Marker für den spezifischen Nachweis von Kollagen genutzt werden (Z. Gunja-Smith et al., Biochem. J. 197 (1981), 759-762). Beim Abbau von extrazellulärem Kollagen gelangen Hydroxylysylpyridinolin- oder Lysylpyridinolinderivate, welche Peptidseitenketten enthalten oder freie Pyridinolin-Derivate mit Lysyl- oder Hydroxylysylresten, wie in der WO 91/10141 beschrieben, in Körperflüssigkeiten wie Blut oder Urin. Der Nachweis dieser Verbindungen in Körperflüssigkeiten ergibt daher Hinweise auf den Abbau von extrazellulärem Kollagen, wie er z.B. bei der Osteoporose sowie infolge von Tumoren des Knochengewebes auftritt. Zum Nachweis von solchen Hydroxylysyl- oder Lysylpyridinolinen mit Peptidseitenketten wurden in der WO 89/12824 monoklonale Antikörper beschrieben, welche durch Immunisierung mit entsprechenden quervernetzten Kollagenfragmenten erhalten wurden, die aus dem Urin isoliert werden können. Auch bei dem in WO 91/08478 beschriebenen Verfahren erfolgt der Nachweis von Kollagen über einen Antikörper gegen natürliche, d.h. in vivo erzeugte quervernetzte Abbauprodukte von Kollagen.

Ein Nachteil dieser aus natürlichen Quellen isolierten Peptide ist, daß keine sichere Quelle für eine reproduzierbare Herstellung der Antigene oder Bindepartner im Test vorhanden ist. Ein weiterer Nachteil der aus natürlichen Quellen isolierten Peptide ist die Gefahr einer Kontamination mit infektiösem Material.

Definierte Antigene können z.B. durch chemische Synthese eines Peptids erhalten werden, welches einem Epitop des Antigens entspricht. Werden hierfür kleine Peptide mit einem Molekulargewicht von etwa 700 - 1500 D verwendet, so ist die Bindung an ein Trägermolekül erforderlich, um ein Antigen mit immunogener Wirkung zu erhalten. Durch die Bindung an das Trägermolekül darf dabei die Struktur des Epitops nicht verändert werden. Die Kupplung an das Trägermolekül erfolgt daher bislang bevorzugt an den Enden der Peptidkette in ausreichendem Abstand von dem vermuteten Epitopbereich (Laboratory Technics in Biochemistry and Molecular Biology, Synthetic Polypeptides as Antigens, Editors R.H. Burdon und P.H. van Knippenberg, Elsevier, Amsterdam, New York, Oxford 1988, Seiten 95-100).

Ein Problem bei der chemischen Synthese eines definierten Antigens, das einem natürlichen Abbauprodukt von quervernetztem Kollagen entspricht, stellt die aus der Quervernetzung resultierende Hydroxylysyl- oder LysylpyridinolinStruktur dar, deren chemische Synthese aufwendig ist.

Aufgabe der Erfindung war es, einen Immunoassay zum Nachweis von Kollagen Typ I oder Kollagenfragmenten davon zur Verfügung zu stellen.

Bisher wurde immer davon ausgegangen, daß es zum Nachweis der Kollagen oder Kollagenabbauprodukte in einer Probe notwendig sei, die Quervernetzungsstrukturen an sich oder sogenannte quervernetzte Peptide, die durch die Quervernetzung der Hydroxylysyl- oder Lysylreste zustandekommen, nachzuweisen, da diese Hydroxylysyl- oder Lysylpyridinolinstruktur charakteristisch für Kollagen ist. Beispiele für solche Nachweismethoden sind in der WO 89/12824, WO 91/08478, WO 89/04491 und der WO 91/10141 beschrieben.

In der EP-A-0 298 210 wird ein Verfahren zur selektiven immunologischen Bestimmung von intaktem Prokollagen Peptid (Typ III) und Prokollagen (Typ III) in Körperflüssigkeiten offenbart.

Die WO 90/08195 werden Oligopeptide, die Segmenten von humanem Typ IX Kollagen entsprechen, als Antigene zur Produktion von monoklonalen Antikörpern, die an humanes Typ IX Kollagen und Fragmente davon binden, verwendet.

EP-A-0 505 210 beschreibt einen Test zur Bestimmung des Abbaus von Typ I Kollagen. Es wird ein Antikörper genutzt, der durch Immunisierung mit einem carboxyterminalen quervernetzten Telopeptid aus Typ I Kollagen hergestellt wurde.

EP-A-0 465 104 beschreibt eine Methode zur immunologischen Bestimmung des carboxyterminalen Propeptids von Typ I Prokollagen.

WO 91/09113 offenbart synthetische Polypeptide mit Typ IV Kollagen Aktivität.

EP-A-0 316 306 beschreibt einen Test zum Nachweis von glykosyliertem Hämoglobin. Eine Denaturierung durch chemische oder physikalische Maßnahmen verbessert die Exposition der linearen Epitope für die Antikörperbindung. Ebenso wird bei der EP-A-0 121 303 offenbart, daß die Behandlung eines Delta Antigens mit einem Detergenz die Antikörperbindung beeinflußt.

In der WO 93/15107 werden Peptide des amino-terminalen Propeptids von al Typ 1 Kollagen in einem Test zum Monitoren der Knochenbildung benutzt.

Keines dieser Dokumente offenbart einen Immunoassay zum Nachweis von Kollagen Typ I oder Kollagenfragmenten davon in einer Probe zum Nachweis einer möglichen Osteolyse, worin ein Bindepartner, der ein synthetisches lineares Peptid erkennt, verwendet wird, welches Peptid einer Sequenz des nichthelicalen C- oder N-terminalen Bereiches von Kollagen Typ I entspricht und worin die Probe denaturiert wird.

Es wurde nun überraschenderweise gefunden, daß es zur Lösung der oben genannten Aufgabe ausreicht, ein definiertes Antigen, einen Bindepartner oder ein Standardmaterial zu verwenden, das. ein synthetisches lineares Peptid enthält, das einer Sequenz des nichthelikalen linearen C- oder N-terminalen Bereiches von Kollagen Typ I entspricht. Die Vorteile bei Verwendung von synthetischen linearen Peptiden zur Verwendung als Bindepartner in Immunoassays, als Standardmaterial oder als Immunogen bei der Antikörper-Herstellung liegen darin, daß diese Peptide, im Gegensatz zu Peptiden aus natürlichen Quellen, in genau definierter Struktur reproduzierbar hergestellt werden können. Zudem zeigt ein Immunoassay, in dem solche kurzen synthetischen Peptide eingesetzt werden, eine geringere Störanfälligkeit.

Gegenstand der Erfindung ist daher ein kompetitiver Immunoassay zum Nachweis von Kollagen Typ I oder Kollagenfragmenten in einer Probe, der dadurch gekennzeichnet ist, daß ein Bindepartner, der ein synthetisches lineares Peptid enthält, das einer Sequenz des nichthelikalen linearen C- oder N-terminalen Bereiches von Kollagen Typ I entspricht, mit einem Antikörper, der das synthetische lineare Peptid zu binden vermag, und der Probe inkubiert und die Bindung des Antikörpers an den Bindepartner in geeigneter Weise bestimmt wird.

Es hat sich als besonders vorteilhaft erwiesen, die Probe beziehungsweise das in dieser Probe enthaltene Kollagen oder die Kollagenfragmente zu denaturieren und die Epitope so der Bindung des Antikörpers besser zugänglich zu machen. Am geeignetsten wird die Probe vor der Inkubation mit dem Antikörper mit dem Denaturierungsmittel für Proteine, die dem Fachmann bekannt sind, vorbehandelt. Um die Beeinträchtigung der immunologischen Reaktion zwischen der Probe und den spezifischen Antikörpern möglichst gering zu halten oder gänzlich zu vermeiden, wird die denaturierte Probe vor der Inkubation mit dem Antikörper bevorzugt noch verdünnt. Als Denaturierungsmittel sind alle dem Fachmann bekannten Mittel hierfür geeignet. Kaliumrhodanid (KSCN) in einer Konzentration von 2 - 6 M und Tetradecyltriäthylammoniumbromid (TTAB) in einer Konzentration von 0,5 - 2 M haben sich als besonders geeignet zur Denaturierung erwiesen.

Durch die Einführung eines Denaturierungsschrittes der Probe konnten Antikörper, die gegenüber nicht-denaturierten Proben nur eine unwesentliche Bindung aufzeigten, zusätzlich genutzt werden, das heißt, sie binden sehr gut eine denaturierte Probe. Durch die Einführung dieses Denaturierungsschrittes stehen daher mehr Antikörper für einen diagnostischen Nachweis von Kollagen oder Kollagenfragmenten zur Verfügung.

Als synthetische lineare Peptide sind alle zusammenhängenden Aminosäuresequenzen des nichthelikalen C-oder N-terminalen Bereichs von Kollagen geeignet. Diese Bereiches sind aus Chu et al., Nature 310, 337-340 (1984), Click et al., Biochemistry 9, 4699-4706 (1970), Morgan et al., J. Biol. Chem. 245, 5042-5048 (1970) und Bernard et al., Biochemistry 22, 5213-5223 (1983) bekannt. Bevorzugt werden Peptide aus 5 bis 25 Aminosäuren, besonders bevorzugt aus 8 bis 20 Aminosäuren, verwendet. Dabei ist es nicht notwendig, daß die Sequenz den Bereich der Quervernetzung umfaßt. Sie kann aber sehr wohl ebenfalls mit diesem Bereich überlappen. In keinem Fall liegt aber in dem synthetischen Peptid eine Hydroxylysyl- oder Lysylpyridinolin-Quervernetzung vor. Am geeignetsten haben sich die synthetischen Peptide aus dem C-terminalen Bereich von Kollagen erwiesen, da der nichthelikale C-terminale Bereich größer als der nichthelikale N-terminale Bereich von Kollagen ist. In diesem Bereich stehen somit mehr potentielle Epitope als in den N-terminalen Bereich zur Verfügung. Peptide mit der in SEQ ID NO 1, 2, 3 oder 4 gezeigten Sequenz aus dem C-terminalen Bereich der α1-Kette von Kollagen sind besonders geeignet.

Die Konzentration von Abbauprodukten von Kollagen ist ein wichtiger diagnostischer Marker für das Ausmaß einer Osteolyse. Mit Hilfe der synthetischen linearen Peptide ist es möglich, einen kompetitiven Immunoassay zum Nachweis von Kollagen oder Kollagenfragmenten durchzuführen. Es hat sich überraschenderweise gezeigt, daß diese Peptide sehr gut mit den Kollagenfragmenten, die in natürlichen Proben wie Plasma, Serum oder Urin vorkommen, um Antikörper gegen diese Kollagenfragmente konkurrieren und damit einen kompetitiven Test ermöglichen. Solche Antikörper gegen Kollagen oder Kollagenabbauprodukte sind kommerziell erhältlich, beispielsweise in dem Telopeptide ICTP [¹²⁵J] Radioimmunoassay Kit der Firma Orion Diagnostica, Finnland. Sie können aber bevorzugt erfindungsgemäß mittels des synthetischen linearen Peptids hergestellt werden.

Zum Einsatz in einem kompetitiven Immunoassay kann das synthetische lineare Peptid direkt als Bindepartner, der an eine Festphase gebunden ist, benutzt werden oder aber gekoppelt an eine zweite Komponente. Die Kopplung an die zweite Komponente erfolgt bevorzugt über die N- und C-terminale Aminosäuren des linearen Peptids. Gegebenenfalls kann zwischen dem Peptid und der zweiten Komponente noch ein Spacer eingefügt werden. Die zweite Komponente kann beispielsweise dazu dienen, das Peptid indirekt an eine Festphase zu koppeln. Beispiele hierfür sind dem Fachmann bekannt. Bevorzugt wird das Peptid an Rinderserumalbumin gekoppelt und das Kopplungsprodukt adsorptiv an eine Festphase zum Beispiel ein Kunststoffröhrchen gebunden. Das Peptid kann auch an Biotin kovalent gebunden werden. Die Anheftung an die Festphase erfolgt dann über die Bindung an Avidin oder Streptavidin, das seinerseits an die Festphase gebunden wurde. Die zweite Komponente kann auch als Träger für mehrerer Peptide dienen, zum Beispiel in einem kompetitiven turbidimetrischen Inhibierung-Immunoassay (TINIA), wobei mehrere Peptide an beispielsweise Albumin, Immunglobuline, β-Galaktosidase, Polymere wie Polylysin oder Dextranmoleküle wie in der EP-A-0 545 350 beschrieben oder Partikel wie Latex gekoppelt sind. Vorzugsweise werden 30 bis 40 Peptidmoleküle je Trägermolekül gekoppelt. Das Peptid kann auch an eine Komponente, die eine Markierung darstellt, gekoppelt sein. Beispiele für all diese Testvarianten sind dem Fachmann bekannt.

Bei der Testführung kann der Antikörper gleichzeitig oder sequentiell mit der Probe sowie dem Bindepartner, der das synthetische lineare Peptid enthält, inkubiert werden. Anschließend wird in üblicher Weise die Menge des gebundenen oder nichtgebundenen Antikörpers bestimmt. Beispielsweise kann der verwendete Antikörper selbst markiert sein und diese Markierung direkt als Maß für den gebundenen oder ungebundenen Antikörper dienen. Es kann auch ein zweiter markierter Antikörper verwendet werden, der gegen den gebundenen oder nicht gebundenen Antikörper, beispielsweise gegen das Fc-Teil dieses Antikörpers, gerichtet ist. Als kompetitive Testvarianten zur Bestimmung der Menge an gebundenem oder ungebundenem Antikörper können beispielsweise Agglutinationstests, wie TINIA, oder FPIA- (Fluoreszenz-Polarisations-Immunoassay) (W. Dandliker et al., J.Exp. Med. 122 (1965), 1029), EMIT- (Enzyme Multiplied Immunoassay) (Gunzer et al., Kontakte III (1980), 3 - 11) und CEDIA-Prinzipien (Henderson et al., Clinical Chemistry 32 (1986), 1637 - 1641) dienen. Die erfindungsgemäßen Peptide haben sich als besonders geeignet für die Verwendung als definierte Bindepartner zur Kompetition mit der Probe um die Bindung an die Antikörper erwiesen. Besonders bevorzugt sind die synthetischen linearen Peptid mit der in SEQ ID NO 1, 2, 3 oder 4 gezeigten Sequenz.

Nach der Bestimmung des Ausmaßes der Bindung des Antikörpers an den Bindepartner, das ein Maß für die Menge an Antigen in der Probe darstellt, kann die genaue Menge an Antigen in der Probe in üblicher Weise durch Vergleich mit in gleicher Weise behandeltem Standard ermittelt werden.

Als Standard können Kollagenabbauprodukte isoliert aus natürlichem Material verwendet werden. Diese zeichnen sich jedoch naturgemäß durch eine bestimmte Schwankung aus. Geeigneter erwiesen hat sich als Standardmaterial ein Antigen, das das erfindungsgemäße synthetische lineare Peptid enthält. Das Antigen des Standards kann dabei lediglich aus diesem Peptid bestehen oder aber aus diesem Peptid, das an einen geeigneten Träger gekoppelt ist, der beispielsweise zur besseren Wasserlöslichkeit des Peptids dient. Zur Herstellung des Standardmaterials aus Peptid und Träger wird das lineare Peptid, das einer Sequenz des nichthelikalen C- oder N-terminalen Bereichs von Kollagen entspricht, synthetisiert und über seine N- oder C-terminale Aminosäure durch geeignete Kopplungsmethoden an das Trägermolekül gebunden. Es können ein oder mehrere Peptide pro Trägermolekül gebunden werden. Gegebenenfalls kann die Kopplung über einen Spacer erfolgen. Für bestimmte Zwecke, wie beispielsweise für Agglutinationstests, kann es von Vorteil sein, mehrere erfindungsgemäße Peptide unterschiedlicher Sequenz an ein Trägermolekül zu binden, vor allem falls polyklonale Antikörper im Test eingesetzt werden, die nicht mit Hilfe des erfindungsgemäßen Antigens hergestellt wurden und damit meist mehrere Epitope erkennen.

Als Antikörper in dem kompetitiven Immunoassay können die bereits bekannten Antikörper gegen Kollagenabbauprodukte verwendet werden. Geeignet sind insbesondere Antikörper, die mit Hilfe eines Antigens, das das erfindungsgemäße lineare synthetische Peptid enthält, gewonnen wurden.

Zur Immunisierung werden die linearen synthetischen Peptide, die einer oder mehreren Sequenzen des nichthelikalen C- oder N-terminalen Bereichs von Kollagen entsprechen, bevorzugt an ein geeignetes Trägerprotein, wie zum Beispiel Keyhole Limpet Hemocyanine, Rinderserumalbumin oder Edestin gebunden werden.

Zur Herstellung dieser Antigene oder Immunogene werden zunächst die linearen Peptide in üblicher Weise chemisch synthetisiert. Anschließend erfolgt die Kopplung der synthetischen Peptide über die N-terminale Aminogruppe mittels Maleinimidohexansäure-N-hydroxysucciminidester an die oben genannten Trägerproteine. Es hat sich überraschenderweise gezeigt, daß zur Herstellung von Antikörpern, die für die kompetitive Test führung geeignet sind, synthetische lineare Peptide mit der in SEQ ID NO 1, 2, 3 oder 4 gezeigten Sequenz besonders geeignet sind.

Mit den Antigenen, die ein synthetisches lineares Peptid enthalten, das einer Sequenz des nichthelikalen C- oder N-terminalen Bereichs von Kollagen entspricht, ist es möglich, Antikörper zu erhalten, welche nicht nur das Peptid sondern auch die in Körperflüssigkeiten vorkommenden Abbauprodukte von Kollagen erkennen.

Monoklonale Antikörper gegen Kollagen oder Kollagenfragmente können durch Immunisierung mit einem erfindungsgemäßen Antigen, Immortalisierung der Milzzellen der immunisierten Tiere, Klonierung derjenigen immortalisierten Milzzellen, welche den gewünschten Antikörper produzieren und Isolierung des Antikörpers aus den klonierten Zellen oder dem Kulturüberstand dieser Zellen, hergestellt werden.

Die Immunisierung erfolgt in den hierfür üblicherweise verwendeten Tieren, vorzugsweise werden Mäuse oder Kaninchen verwendet.

Die Immortalisierung der Milzzellen der immunisierten Tiere erfolgt nach dem Fachmann geläufigen Verfahren, wie z.B. der Hybridomtechnik (Köhler und Milstein, Nature 256 (1975), 495 - 497) oder durch Transformation mit dem Epstein-Barr-Virus (EBV-Transformation). Zum Nachweis derjenigen immortalisierten Zellen, die den gewünschten Antikörper produzieren, wird eine Probe des Kulturüberstands in einem üblichen Immunoassay mit dem zur Immunisierung verwendeten erfindungsgemäßen Antigen inkubiert und untersucht, ob ein Antikörper an dieses Antigen bindet.

Diese polyklonalen und monoklonalen Antikörper reagieren nicht nur mit dem zur Immunisierung verwendeten erfindungsgemäßen Hapten, sondern gut mit Kollagen, sowie mit den in Körperflüssigkeiten gefundenen natürlichen Abbauprodukten von Kollagen. Das in der Probe vorkommende Kollagen oder dessen Fragmente wird denaturiert, wodurch die Bindung der erfindungsgemäßen Antikörper in den meisten Fällen erheblich verbessert wird.

Die Antikörper können daher in Nachweisverfahren zur Bestimmung von Kollagen oder Kollagenfragmenten verwendet werden.

Gegenstand der Erfindung ist daher ein Immunoassay zum Nachweis von Kollagen oder Kollagenfragmenten in einer Probe unter Verwendung mindestens eines Antikörpers, der ein synthetisches lineares Peptid erkennt, das einer Sequenz des nichthelikalen C- oder N-terminalen Bereiches von Kollagen Typ I entspricht, der dadurch gekennzeichnet ist, daß die Probe denaturiert wird. Am geeignetsten erwiesen hat sich der zuvor beschriebene kompetitive Immunoassay unter weiterer Verwendung der beschriebenen linearen Peptide. Die Verwendung der beschriebenen Antikörper ist jedoch keineswegs auf den kompetitiven Immunoassay beschränkt. Die Antikörper können ebenfalls in anderen Testformaten, beispielsweise einem Sandwich-Immunoassay eingesetzt werden. Als zweiter Antikörper in diesem Immunoassay können beispielsweise Antikörper des Standes der Technik eingesetzt werden. Es muß lediglich darauf geachtet werden, daß die beiden Antikörper nicht miteinander um die gleiche Bindungsstelle konkurrieren.

Gegenstand der Erfindung ist ferner eine Testkombination zum Nachweis von Kollagen oder Kollagenfragmenten Typ I in einer Probe enthaltend in einem ersten Reagenz ein Protein-Denaturierungsmittel und getrennt davon in einem zweiten Reagenz einen Antikörper, der ein synthetisches lineares Peptid erkennt, das einer Sequenz des nichthelikalen C- oder N-terminalen Bereiches von Kollagen Typ I entspricht. Die Reagenzien liegen entweder in Form wässriger, vorzugsweise gepufferter, Lösungen vor, wobei als Puffer alle üblichen, dem Fachmann bekannten Puffer verwendet werden können, die die immunologische Reaktion nicht stören, oder aber in Form trockener, vorzugsweise lyophilisierter Gemische, die durch Zugabe eines geeigneten Lösungsmittels wie Wasser rekonstituiert werden können.

Zusätzlich können noch andere übliche Testzusätze wie Entstörsubstanzen, Proteine oder Detergenzien enthalten sein. Bevorzugt ist in der Testkombination noch zusätzlich ein synthetisches lineares Peptid, das einer Sequenz des nichthelikalen C- oder N-terminalen Bereiches von Kollagen entspricht, als Bindepartner des Antikörpers enthalten. Das synthetische lineare Peptid kann entweder an einen Träger direkt gekoppelt oder aber an eine zweite Komponente gekoppelt sein, die die Bindung an die feste Phase vermittelt. Ebenfalls kann das Peptid an eine Komponente, die eine Markierung darstellt, gekoppelt sein.

Weiterhin kann in einem dritten Reagenz ein Standard zur Erstellung einer Standard- oder Eichkurve enthalten sein, der ein Antigen enthält, das ein synthetisches Peptid, das einer Sequenz des nichthelikalen C- oder N-terminalen Bereiches von Kollagen entspricht, enthält.

Die Erfindung wird durch die folgenden Beispiele in Verbindung mit den Sequenzprotokollen näher erläutert.
- SEQ ID NO 1: zeigt die Sequenz eines erfindungsgemäßen Peptids aus 9 Aminosäuren, wobei Xaa eine beliebige Aminosäure bedeutet.
- SEQ ID NO 2: zeigt die Sequenz eines erfindungsgemäßen Peptids aus 16 Aminosäuren.
- SEQ ID NO 3: zeigt die Sequenz eines erfindungsgemäßen Peptids aus 10 Aminosäuren.
- SEQ ID NO 4: zeigt die Sequenz eines erfindungsgemäßen Peptids aus 13 Aminosäuren.

### Beispiel 1

### Peptidsynthesen

Die eine Teilsequenz der Aminosäurensequenz von Kollagen aufweisenden Peptide der in den Sequenzprotokollen SEQ ID NO 2 und 3 gezeigten Sequenzen werden mittels Fluorenylmethyloxycarbonyl (Fmoc)-Festphasenpeptidsynthese an einem a) Labortec SP 640 Peptide Synthesizer bzw. b) Zinsser Analytic SMPS 350 Peptidsynthesizer hergestellt.
a) Herstellung von Acetyl-Ser-Ala-Gly-Phe-Asp-Phe-Ser-Phe-Leu-Pro-Gln-Pro-Pro-Gln-Glu-Lys-Amid (SEQ ID NO 2)
   In der angegebenen Reihenfolge werden jeweils 4,0 Äquivalente von folgenden Fmoc-Aminosäurederivaten eingesetzt:
   - Lys: mit tert. Butyloxycarbonyl-Schutzgruppe
   - Glu: mit tert. Butylester-Schutzgruppe
   - Gln: ohne Seitenketten-Schutzgruppe
   - Pro: ohne Seitenketten-Schutzgruppe
   - Pro: ohne Seitenketten-Schutzgruppe
   - Gln: ohne Seitenketten-Schutzgruppe
   - Pro: ohne Seitenketten-Schutzgruppe
   - Leu: ohne Seitenketten-Schutzgruppe
   - Phe: ohne Seitenketten-Schutzgruppe
   - Ser: mit tert. Butylether-Schutzgruppe
   - Phe: ohne Seitenketten-Schutzgruppe
   - Asp: mit tert. Butylester-Schutzgruppe
   - Phe: ohne Seitenketten-Schutzgruppe
   - Gly: ohne Seitenketten-Schutzgruppe
   - Ala: ohne Seitenketten-Schutzgruppe
   - Ser: mit tert. Butylether-Schutzgruppe
   - Acetyl: Essigsäureanhydrid

   Die Aminosäuren oder Aminosäurederivate werden in N-Methylpyrrolidon gelöst.
   Das Peptid wird an 3 g 4-(2',4'-Dimethoxyphenyl-Fmocaminomethyl)-phenoxy-Harz (Tetrahedron Letters 28 (1987), 2107) mit einer Beladung von 0,87 mmol/g synthetisiert (JACS 95 (1973), 1328). Die Kupplungsreaktionen werden bezüglich Fmoc-Aminosäurederivat mit 4,4 Äquivalenten Dicyclohexylcarbodiimid und 4,8 Äquivalenten N-Hydroxybenzotriazol in Dimethylformamid als Reaktionsmedium während 60 Minuten durchgeführt. Der Kupplungserfolg wird mittels Kaiser-Test (Anal. Biochem. 34 (1970), 595) an dem mit Isopropanol gewaschenen Syntheseharz kontrolliert. Sofern sich hierbei ein noch nicht vollständiger Umsatz ergibt, wird durch Nachkuppeln gemäß den oben angegebenen Bedingungen, der Umsatz vervollständigt. Nach jedem Syntheseschritt wird die Fmoc-Gruppe mittels 20%-igem Piperidin in Dimethylformamid in 20 Minuten abgespalten. Die Harzbeladung wird mittels UV-Adsorbtion der freigesetzten Fulven-Gruppe nach jeder Piperidinbehandlung ermittelt. Nach der Synthese beträgt die Beladung noch 0,68 mmol/g.
   Die Freisetzung des Peptids vom Syntheseharz und die Abspaltung der säurelabilen Schutzgruppen erfolgt mit 80 ml Trifluoressigsäure, 5 ml Ethandithiol, 2,5 g Phenol, 2,5 ml m-Kresol und 5 ml Wasser in 60 Minuten bei Raumtemperatur.
   Die Reaktionslösung wird anschließend im Vakuum eingeengt. Der Rückstand wird in Diisopropylether aufgenommen, 1/2 - 2 Stunden kräftig gerührt und dann abfiltriert. Das Material wird dann mittels einer Gelpermeationschromatographie an Sephadex G15 vorgereinigt, wobei als Elutionsmittel 0,5 %-ige Essigsäure verwendet wird. Das erhaltene Rohmaterial wird anschließend abfiltriert und mittels einer präparativen HPLC an Nucleosil RP18 (Säule 40 mm x 250 mm 300 Å, 5 µm) über einen Gradienten von 100 % Puffer A (Wasser, 0,1 % Trifluoressigsäure) zu 100 % Puffer B (60 % Acetonitril, 40 % Wasser, 0,1 % Trifluoressigsäure) in 120 Minuten isoliert. Die Identität des eluierten Materials wird mittels Fast-Atom-Bombardment-Massenspektormetrie (FAB-MS) geprüft.
**b) Herstellung von Ala-Gly-Phe-Asp-Phe-Ser-Phe-Leu-Pro-Gln (SEQ ID NO 3)**
   Das Peptid wurde an 30 mg 4-(2',4'-Dimethoxyphenyl-Fmocaminomethyl)phenoxy-Harz SA 5030 der Firma Advanced Chemtech mit einer Beladung von 0,47 mmol/g hergestellt. Von folgenden Fmoc-Aminosäure-Derivaten wurden zweimal je 140 µMol zusammen mit je 140 µMol 1-Hydroxybenzotriazol in Dimethylformamid DMF und 10 µMol N,N-Diisopropylcarbodiimid in DMF an das aufzubauende festphasengebundene Peptid angekoppelt:

Die Kupplungszeiten betrugen 30 und 40 Minuten. Die Abspaltzeit betrug 20 Minuten und wurde mit einer Lösung von 50% Piperidin im DMF durchgeführt. Die Waschschritte wurden mit DMF achtmal nach jedem Reaktionsschritt durchgeführt. Die Freisetzung des Peptids erfolgte durch Behandlung des vom Lösungsmittel abfiltrierten und mit Dichlormethan und Methanol gewaschenen Harz mit 1 ml einer Lösung von 90% Trifluoressigsäure, 3% Thioanisol, 3 % Ethandithiol und 3 % Thiokresol innerhalb von 20 Minuten und 140 Minuten. Das Produkt wurde durch Zugabe vom 15 ml kaltem Diisopropylether zum vereinigten Filtrat ausgefällt und durch Filtration isoliert. Der Rückstand wurde in 50%iger Essigsäure gelöst und lyophilisiert. Es wurden 8 mg weißes Lyophilisat einer Reinheit von 79% laut HPLC erhalten. Die Identität wurde mittels FAB-Massenspektroskopie bestätigt.

Das Peptid mit der Sequenz SEQ ID NO 4 Cys-Gly-Ser-Ala-Gly-Phe-Asp-Phe-Ser-Phe-Leu-Pro-Gln wurde in analoger Weise hergestellt.

### Beispiel 2

### Aktivierung von Peptiden

Das gemäß Beispiel 1a) hergestellte Peptid wird durch Acylierung mit Maleinimidohexanoyl-N-hydroxysuccinimid (MHS) aktiviert. Dazu wird 0,1 mmol des Peptids in 20 ml 0,1 mol/l Kaliumphosphatpuffer pH 7,5 gelöst, mit einer Lösung von 0,1 mmol MHS in 6 ml Dioxan versetzt und 20 min. bei 20°C gerührt. Anschließend wird der pH-Wert mit Eisessig auf pH 4 eingestellt und das Reaktionsgemisch sofort lyophilisiert. Das Lyophilisat wird in 5 ml Wasser gelöst und über präparative HPLC an einer Waters Delta-Pak® C18-Säule (100 Å, 15 µm 50 x 300 mm) über einen Elutionsgradienten von 100 % A (Wasser 0,1 % Trifluoressigsäure) zu 100 % B (99,9 % Acetonitril 0,1 % Trifluoressigsäure) aufgereinigt.

### Beispiel 3

### Herstellung von Immunogenen durch Kopplung von aktivierten Peptiden an Trägerproteine

Beschrieben wird die Kopplung von aktivierten Peptiden an Keyhole Limpet Hemocyanine (KLH), Rinderserumalbumin (RSA) und β-Galaktosidase (βGal). Zur Kopplung der nach Beispiel 2 mit MHS aktivierten Peptide ist es erforderlich, daß das Trägerprotein freie SH-Gruppen trägt. βGal weist diese natürlicherweise bereits auf, erfordert also keine weitere Vorbehandlung. Bei KLH und RSA werden die NH₂-Gruppen der ε-Aminoseitenkette von Lysinresten durch Behandlung mit N-Succinimidyl-S-acetylthiopropionat (SATP) derivatisiert und dadurch in SH-Gruppen umgewandelt.

Man erhält somit ein Trägerprotein das eine gegenüber dem nativen Zustand erhöhte Anzahl von SH-Gruppen aufweist. Hierfür wird zu einer Lösung aus 1,39 g KLH in 500 ml 0,1 mol/l Kaliumphosphatpuffer pH 8,5 innerhalb von 20 min. 113,51 mg SATP (in 10 ml Dioxan gelöst) zugetropft. Nach 30 min. Rühren bei 20°C wird der pH-Wert der Reaktionslösung mit 0,1 mol/l Natronlauge auf pH 8,5 nachgestellt und weitere 24 Stunden gerührt. Die Lösung wird anschließend mit Hilfe einer Amiconzelle (Membran YM10) auf 100 ml aufkonzentriert, 3 x 24 Stunden gegen je 3 1 0,1 mol/l Kaliumphosphatpuffer pH 8,5/0,05 mol/l Natriumchlorid dialysiert und anschließend lyophilisiert.

Zur Abspaltung der S-Acetylschutzgruppe werden 481 mg des KLH-SATP Lyophilisats in 20 ml 0,1 mol/l Kaliumphosphatpuffer pH 8,5/0,05 mol/l Natriumchlorid gelöst, mit 0,5 ml frisch zubereiteter 1 mol/l Hydroxylaminlösung versetzt und 90 min. bei 20°C gerührt.

7,23 mol des aus Beispiel 2 erhaltenen aktivierten Peptids in 4 ml Wasser werden zu dem derivatisierten Trägerprotein gegeben und 20 Stunden bei 20°C gerührt. Anschließend wird die trübe Lösung 2 x gegen 1 l 0,1 mol/l Kaliumphosphatpuffer pH 8,5/0,05 mol/l Natriumchlorid dialysiert. Das Dialysat wird zentrifugiert, der klare Überstand abdekantiert und lyophilisiert.

### Beispiel 4

### Herstellung polyklonaler Antikörper gegen lineare Kollagenfragmente

Je 5 Schafe wurden in an sich bekannter Weise mit dem Immunogen aus Beispiel 3 immunisiert. Die Immunogene enthielten das Peptid mit der in SEQ ID NO 2 genannten Sequenz, das den Aminosäuren Nr. 892 bis 907 in der Sequenz der α-Kette von Kollagen Typ I entspricht. Als Trägerprotein diente KLH bzw. β-Galaktosidase. Die Tiere wurden in monatlichen Abständen mit den Immunogenen in kompletten Freundschen Adjuvans immunisiert. Die Dosis betrug 500 µg je Tier und Immunisierung. Vier Monate nach Erstimmunisierung wurden Blutproben entnommen und die erhaltenen Antikörper auf Reaktion mit Kollagenfragmenten geprüft.

### ELISA zum Nachweis der Reaktion der Antiseren mit Kollagenfragmenten

Folgendes Material und Reagenzien wurden verwendet:
Mikrotiterplatten Maxisorp® F96, Firma Nunc
- Beschichtungspuffer:: 50 mM Natriumcarbonat pH 9,6
0,1 % NaN₃
- Inkubationspuffer:: 10 mM Natriumphosphat pH 7,4
0,1 % Tween® 20
0,9 % NaCl
1 % Rinderserumalbumin
- Substratlösung:: ABTS®, Boehringer Mannhein GmbH, Katalog
Nr. 857424.
Zur Verstärkung des Signals wurde der
Lösung 2 mg/ml Vanillin zugesetzt.
- Waschlösung:: 0,1 % Tween® 20
0,9 % NaCl

Die Vertiefungen der Titerplatten wurden mit je 100 µl einer Lösung gefüllt, die 10 µg/ml Kollagenfragmente in Beschichtungspuffer enthielt. Die Kollagenfragmente wurden entsprechend den Angaben in EP-A-0 505 210 durch Protease-Verdauung von humanem Kollagen aus Knochen hergestellt. Nach einer Stunde Inkubation bei Raumtemperatur unter Schütteln wurde dreimal mit Waschlösung gewaschen.

Die Antiseren wurden mit Inkubationspuffer 1 : 4000 verdünnt und je 100 µl in den Vertiefungen der Mikrotiterplatte eine Stunde unter Schütteln bei Raumtemperatur inkubiert. Anschließend wurden die Vertiefungen dreimal mit Waschlösung gewaschen.

Ein Konjugat aus Meerettich-Peroxidase mit Kaninchen-Antikörpern gegen den Fc-Teil von Schaf-IgG wird im Inkubationspuffer bis zur Konzentration von 12,5 mU/ml verdünnt und die Näpfe der Mikrotiterplatte mit je 100 µl davon beschickt. Nach einer Stunde Inkubation unter Schütteln bei Raumtemperatur werden die Titerplatten dreimal mit Waschlösung gewaschen.

100 µl Substratlösung werden hinzugefügt und inkubiert, bis eine Farbentwicklung deutlich wird (10 - 60 Minuten). Die Extinktion wird als Differenzmessung bei 405 und 492 nm erfaßt.

Die Seren der meisten Tiere zeigten eine starke Reaktion mit den Kollagenfragmenten auf der Festphase. Das Serum eines nicht immunisierten Tieres zeigte unter gleichen Bedingungen nur ein schwaches Meßsignal. Die Ergebnisse sind in der Tabelle 1 dargestellt.

**Tabelle 1**

| β-Galaktosidase | | KLH | |
|---|---|---|---|
| Tier-Nr. | Extinktion | Tier-Nr. | Extinktion |
| 1 | 1,05 | 1 | 1,16 |
| 2 | 1,18 | 2 | 2,62 |
| 3 | 1,49 | 3 | 1,28 |
| 4 | 0,48 | 4 | 1,42 |
| 5 | > 2,70 | 5 | 1,81 |

In analoger Weise wurden Mäuse mit Immunogenen nach Beispiel 3 immunisiert und die Antiseren gewonnen. Die drei Antiseren 223/20, 241/13 und 242/2 waren besonders geeignet. Das Antiserum 223/20 entstammte einer Maus, die mit einem Decapeptid entsprechend SEQ ID NO 4 an KLH gekoppelt immunisiert wurde.

Das Antiserum 241/13 entstammte einer Maus, die mit einem Peptid aus 16 Aminosäuren Länge entsprechend SEQ ID NO 2 an KLH gekoppelt immunisiert wurde.

Das Antiserum 242/2 entstammte einer Maus, die mit einem Peptid aus 16 Aminosäuren Länge entsprechend SEQ ID NO 2 an βGal gekoppelt immunisiert wurde.

### Beispiel 5

### Bestimmung von Kollagen, sowie dessen Abbauprodukten in Körperflüssigkeiten über einen kompetitiven Test

Die Vertiefungen einer 96-er Mikrotiterplatte werden gemäß EP-A 0 344 578 mit Streptavidin (100 µl einer Lösung von 1 µg/ml in PBS) bei 4°C über Nacht beschichtet und noch freie unspezifische Bindungsstellen durch Inkubation mit 300µl BSA (Rinderserumalbumin, 10 mg/ml) für 2 Stunden bei Raumtemperatur blockiert.

Das Decapeptid mit der in SEQ ID NO 3 gezeigten Sequenz, das gemäß Beispiel 1b) hergestellt wurde, wird aminoterminal mit D-Biotinyl-ε-amidocapronsäure-N-succinimidester (Boehringer Mannheim, Katalog Nr. 1008960) gemäß Angaben des Herstellers biotinyliert. Das biotinylierte Peptid wird in PBS, 0,05% Tween® 20, 1% BSA in einer Konzentration von 10ng/ml gelöst und durch 1-stündige Inkubation von 100µl je Vertiefung an die mit Streptavidin beschichtete Mikrotiterplatte gebunden. Anschließend wird ungebundenes Peptid durch dreimaliges Waschen mit PBS, 0,05% Tween® 20 entfernt.

Jeweils 150 µl der zu untersuchenden Probe (Serum, Plasma oder ein Standard) werden mit 150 µl des erfindungsgemäßen Antikörpers nach Beispiel 4 für 2 Stunden bei 37°C (oder über Nacht bei 4°C) inkubiert. Jeweils 100µl dieses Ansatzes werden in die Vertiefungen der Mikrotiterplatte zu dem gebundenen Decapeptid gegeben und 60 Minuten bei 37°C inkubiert. Dabei kann nur der nach der Inkubation mit der Probe noch nicht gebundene Antikörperüberschuß des Antiserums an das immobilisierte Decapeptid binden.

Nach dreimaligem Waschen mit PBS/0,05% Tween® 20 wird gebundener Antikörper durch anschließende Inkubation mit einem Kaninchen-Anti-Schaf IgG-POD Konjugat (Boehringer Mannheim GmbH) und ABTS® (1 mg/ml) nachgewiesen.

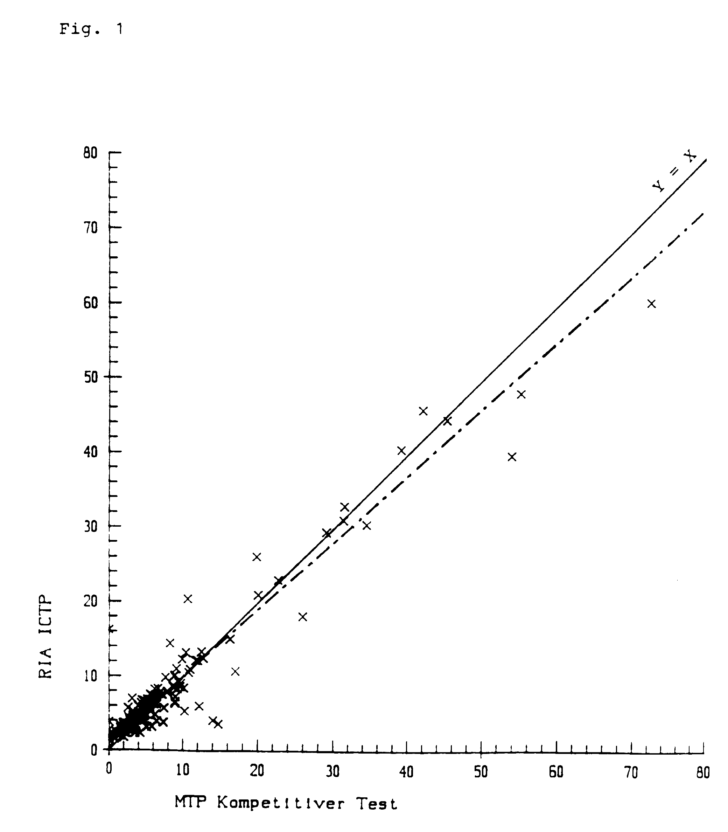

Mit dem erfindungsgemäßen Test (MTP Kompetitiver Test) wurden 164 Patienten-Seren vermessen. Die Ergebnisse wurden zu Daten, welche mit einem Radioimmunoassay (RIA) ermittelt wurden, in Bezug gesetzt. Dieser RIA ICTP (Telopeptide ICTP [¹²⁵J] von Orion Diagnostica, Finnland) basiert auf quervernetzten Kollagenfragmenten, welche durch enzymatischen Verdau und biochemische Verfahren hergestellt und isoliert werden. Aus Figur 1 geht nun hervor, daß das erfindungsgemäße Verfahren Meßwerte erbringt, die gut mit den RIA-Werten korrelieren, das heißt, das erfindungsgemäße Verfahren liefert klinisch relevante Daten. Es wurde ein Korrelationskoeffizient von 0,959 ermittelt.

### Beispiel 6

### Einfluß von denaturierenden Reagenzien auf die Bestimmung eines C-terminalen Peptids aus Kollagen

Wie im Beispiel 5 beschrieben wird das biotinylierte Decapeptid entsprechend SEQ ID NO 3 an mit Streptavidin beschichteten Mikrotiterplatten gebunden.

Alternativ wird ein Fragment von Kollagen, ein C-terminales Telopeptid (CTX), das nach Risteli (1993) hergestellt wurde, an die Oberfläche von Mikrotiterplatten (Nunc, Maxisorb®) adsorbiert. Hierzu werden je 100 µl einer Lösung, die 1µg/ml CTX enthalt, über Nacht bei 4°C in den Vertiefungen einer Mikrotiterplatte inkubiert. Freie unspezifische Bindungsstellen werden durch Inkubation mit 300 µl Rinderserumalbumin-Lösung (10 mg/ml in PBS) für zwei Stunden bei Raumpemperatur blockiert.

Als Probe wird eine CTX-Losung verwendet. Diese wird entweder mit PBS-Lösung allein (Kontrolle), einer PBS-Lösung mit 3 M Kaliumrhodanid (KSCN) oder einer Lösung mit 1 % Tetradecyltriäthylammoniumbromid (TTAB) vorbehandlet. Gleiche Volumen der CTX-Losung und der Puffer-Lösungen werden gemischt und eine Stunde inkubiert. Anschließend wird das Gemisch 1 zu 10 mit einer PBS-Lösung, die 0,05 % Tween® 20® enthielt, verdünnt, da die unverdünnten Denaturierungsmittel die Immunreaktivität der Antikörper beeinträchtigen könnte.

100 µl der so vorbereiteten Proben werden mit 100 µl einer Lösung eines polyklonalen Maus-Antikörpers in der Mikrotiterplatte, die entweder mit dem Decapeptid oder CTX, wie oben beschrieben, beschichtet waren, für eine Stunde bei Raumtemperatur inkubiert. Ungebundene Antikörper wurden durch dreimaliges Waschen mit PBS mit 0,05 % Tween 20® entfernt. Gebundener Antikörper wird mit einem Schaf-Anti-Maus F(ab)-POD-Konjugat (Boehringer Mannheim GmbH, Katalog-Nr. 117 2808) und ABTS® (lmg/ml) nachgewiesen.

Als Antiseren wurden die im Beispiel 4 beschriebenen Antiseren aus Mäusen 223/20, 241/13 und 242/2 benutzt, die vor dem Einsatz 1:2000 in PBS mit 0,05 % Tween 20® verdünnt wurden.

In der Tabelle 2 sind die Ergebnisse (gemessenen Extinktionen) zusammengestellt. CTX in PBS ohne denaturierende Agenzien zeigt nur mit dem Mausserum 242/2 eine signifikante Kompetition. Die Verwendung der denaturierenden Reagenzien KSCN und TTAB bewirkt in allen Fällen eine drastische Erhöhung der Kompetition, auch bei den Antiseren, die ohne denaturierende Agenzien keine deutliche Kompetition zeigten.

**Tabelle 2**

| Probenvorbehandlung | Antiserum 223/20 | Antiserum 241/13 | Antiserum 242/2 |
|---|---|---|---|
| Kontrolle PBS | 1,314 | 0,502 | 0,563 |
| CTX nicht denaturiert | 1,125 | 0,370 | 0,283 |
| Kontrolle KCSN | 1,202 | 0,443 | 0,562 |
| CTX + KSCN | 0,461 | 0,174 | 0,259 |
| Kontrolle TTAB | 0,873 | 0,217 | 0,471 |
| CTX + TTAB | 0,107 | 0,090 | 0,244 |

## Patentansprüche

1. Kompetititver Immunoassay zum Nachweis von KollagenTyp I oder Kollagenfragmenten davon in einer Probe, worin ein Bindepartner, der ein synthetisches lineares Peptid enthält, das einer Sequenz des nichthelikalen C- oder N-terminalen Bereichs von Kollagen Typ I entspricht,
mit einem Antikörper, der das synthetische lineare Peptid zu binden vermag,
und der Probe inkubiert,
und die Bindung des Antikörpers an den Bindepartner in geeigneter Weise bestimmt wird, dadurch gekennzeichnet. daß die Probe denaturiert wird.

2. Verfahren nach Anspruch 1, dadurch gekennzeichnet. daß das synthetische lineare Peptid einer Sequenz des nichthelikalen C-terminalen Bereichs von Kollagen Typ I entspricht.

3. Verfahren nach einem der Ansprüche 1 und 2, dadurch gekennzeichnet, daß das synthetische lineare Peptid aus 5 bis 25 Aminosäuren und bevorzugt aus 8 bis 20 Aminosäuren besteht.

4. Verfahren nach einem der Ansprüche 1 bis 3, dadurch gekennzeichnet, daß das synthetische Peptid der in SEQ ID NO. 1, 2, 3 oder 4 gezeigten Sequenz des nichthelikalen C-terminalen Bereichs von Kollagen Typ I entspricht.

5. Immunoassay nach Anspruch 1, dadurch gekennzeichnet, daß die Denaturierung der Probe vor der Inkubation mit dem Antikörper erfolgt.

6. Immunoassay nach Anspruch 1, dadurch gekennzeichnet, daß als Denaturierungsmittel TTAB oder KCSN verwendet wird.

7. Immunoassay zum Nachweis von Kollagen Typ I oder Kollagenfragmenten davon in einer Probe unter Verwendung mindestens eines Antikörpers, der ein synthetisches lineares Peptid erkennt, das einer Sequenz des nichthelikalen C- oder N-terminalen Bereiches von Kollagen Typ I entspricht, dadurch gekennzeichnet, daß die Probe denaturiert wird.

8. Testkombination zum Nachweis von Kollagen Typ I oder Kollagenfragmenten davon in einer Probe enthaltend in einem ersten Reagenz ein Protein-Denaturierungsmittel und getrennt davon in einem zweiten Reagenz einen Antikörper, der ein synthetisches lineares Peptid erkennt, das einer Sequenz des nichthelikalen C- oder N-terminalen Bereiches von Kollagen Typ I entspricht.

9. Testkombination nach Anspruch 8, dadurch gekennzeichnet, daß zusätzlich ein synthetisches lineares Peptid, das einer Sequenz des nichthelikalen C- oder N-terminalen Bereiches von Kollagen Typ I entspricht, enthalten ist.

## Claims

1. Competitive immunoassay for the detection of type I collagen or collagen fragments thereof in a sample wherein one binding partner which contains a synthetic linear peptide that corresponds to a sequence of the non-helical C-terminal or N-terminal region of type I collagen
is incubated with an antibody that is capable of binding the synthetic linear peptide
and the sample
and the binding of the antibody to the binding partner is determined in a suitable manner, wherein the sample is denatured.

2. Method as claimed in claim 1, wherein the synthetic linear peptide corresponds to a sequence of the non-helical C-terminal region of type I collagen.

3. Method as claimed in one of the claims 1 and 2, wherein the synthetic linear peptide is composed of 5 to 25 amino acids and preferably of 8 to 20 amino acids.

4. Method as claimed in one of the claims 1 to 3, wherein the synthetic peptide corresponds to the sequence of the non-helical C-terminal region of type I collagen shown in SEQ ID NO 1, 2, 3 or 4.

5. Immunoassay as claimed in claim 1, wherein the sample is denatured before incubation with the antibody.

6. Immunoassay as claimed in claim 1, wherein TTAB or KCSN is used as the denaturing agent.

7. Immunoassay for the detection of type I collagen or collagen fragments thereof in a sample using at least one antibody that recognizes a synthetic linear peptide that corresponds to a sequence of the non-helical C-terminal or N-terminal region of type I collagen, wherein the sample is denatured.

8. Test combination for the detection of type I collagen or collagen fragments thereof in a sample containing a protein denaturing agent in a first reagent and separate therefrom in a second reagent an antibody which recognizes a synthetic linear peptide that corresponds to a sequence of the non-helical C-terminal or N-terminal region of type I collagen.

9. Test combination as claimed in claim 8, wherein it additionally contains a synthetic linear peptide that corresponds to a sequence of the non-helical C-terminal or N-terminal region of type I collagen.

## Revendications

1. Dosage immunologique par compétition pour la détection du collagène de type I ou de fragments de celui-ci dans un échantillon, dans lequel on incube un partenaire de liaison, qui contient un peptide linéaire synthétique correspondant à une séquence de la région non-hélicoïdale de la terminaison C ou N du collagène de type I,
avec un anticorps qui confère au peptide linéaire synthétique une capacité de liaison.
et avec l'échantillon,
et on détermine, de manière appropriée, la liaison de l'anticorps au partenaire de liaison, caractérisé par le fait que l'échantillon est dénaturé.

2. Procédé selon la revendication 1, caractérisé par le fait que le peptide linéaire synthétique correspond à une séquence de la région non-hélicoïdale de la terminaison C du collagène de type I.

3. Procédé selon la revendication 1 ou 2, caractérisé par le fait que le peptide linéaire synthétique est constitué par 5 à 25 acides aminés et de préférence, par 8 à 20 acides aminés.

4. Procédé selon l'une quelconque des revendications 1 à 3, caractérisé par le fait que le peptide synthétique correspond une séquence désignée par SEQ ID N° 1, 2, 3 ou 4 de la région non-hélicoïdale de la terminaison C du collagène de type I.

5. Dosage immunologique selon la revendication 1, caractérisé par le fait que la dénaturation de l'échantillon est réalisée avant l'incubation avec l'anticorps.

6. Dosage immunologique selon la revendication 1, caractérisé par le fait qu'en tant qu'agent de dénaturation, on utilise TTAB ou KCSN.

7. Dosage immunologique destiné à la détection du collagène de type I ou de fragments de celui-ci dans un échantillon, utilisant au moins un anticorps qui reconnaît un peptide linéaire synthétique correspondant à une séquence de la région non-hélicoïdale de la terminaison C ou N du collagène de type I, caractérisé par le fait que l'échantillon est dénaturé.

8. Combinaison de test destinée à la détection du collagène de type I ou de fragments de celui-ci dans un échantillon, contenant, dans un premier réactif, un agent de dénaturation de protéine et séparé de celui-ci. dans un second réactif, un anticorps qui reconnaît un peptide linéaire synthétique correspondant à une séquence de la région non-hélicoïdale de la terminaison C ou N du collagène de type I.

9. Combinaison de test selon la revendication 8, caractérisée par le fait qu'elle contient en outre un peptide linéaire synthétique, correspondant à une séquence de la région non-hélicoïdale de la terminaison C ou N du collagène de type I.


### SEQUENZPROTOKOLL

(2) INFORMATION ZU SEQ ID NO: 1:
   (i) SEQUENZ CHARACKTERISTIKA:
      (A) LÄNGE: 9 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 1:
   Xaa Phe Asp Phe Ser Phe Leu Pro Xaa
   1               5
(2) INFORMATION ZU SEQ ID NO: 2:
   (i) SEQUENZ CHARACKTERISTIKA:
      (A) LÄNGE: 16 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 2:
   Ser Ala Gly Phe Asp Phe Ser Phe Leu Pro Gln Pro Pro Gln Glu Lys
   1 5 10 15
(2) INFORMATION ZU SEQ ID NO: 3:
   (i) SEQUENZ CHARAKTERISTIKA:
      (A) LÄNGE: 10 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 3:
   Ala Gly Phe Asp Phe Ser Phe Leu Pro Gln
   1 5 10
(2) INFORMATION ZU SEQ ID NO: 4:
   (i) SEQUENZ CHARAKTERISTIKA:
      (A) LÄNGE: 13 Aminosäuren
      (B) ART: Aminosäure
      (D) TOPOLOGIE: linear
   (ii) ART DES MOLEKÜLS: Peptid
   (xi) SEQUENZBESCHREIBUNG: SEQ ID NO: 4:
   Cys Gly Ser Ala Gly Phe Asp Phe Ser Phe Leu Pro Gln
   1 5 10